# EUROPEAN PATENT SPECIFICATION

(11) **EP 2 388 246 B1**
(45) Date of publication and mention of the grant of the patent: **29.11.2017**
(21) Application number: 11155104.0
(22) Date of filing: 18.02.2011
(51) Int. Cl.: C07C 407/00, A01N 37/16

(54) **Two liquid component system and a process using such system for obtaining a peroxyacid (peracetic acid)**
Flüssiges Zweikomponente-System und ein Verfahren mit solchem System für den Erhalt einer Peroxysäure (Peressigsäure)
Système liquid à deux composants et un procédé utilisant tel système pour l'obtention d'un peroxyacide (acide peracétique)

(30) Priority: 19.02.2010 IT VR20100033
(43) Date of publication of application: 23.11.2011
(73) Proprietor: Myriel s.r.l., 37032 Monteforte d'Alpone (VR) (IT)
(72) Inventor: Preto, Andrea, 37020 Sant'Anna d'Alfaedo - Verona (IT)
(74) Representative: Feltrinelli, Secondo Andrea

(56) References cited:
- EP-A2- 1 371 643
- US-A1- 2002 004 057
- US-A1- 2005 109 981
- US-A1- 2009 043 123

## Description

The present invention concerns a two liquid component system and a process for obtaining very rapidly a concentrated or dilute solution of an organic peroxyacid (e. g. peroxyacetic acid) to be used as such or diluted as a biocide in the field of disinfection, chemical sterilization and in other fields where the organic peroxyacid (e. g. peroxyacetic acid) is used owing to its biocidal (disinfecting and sterilizing), bleaching, descaling detoxifying and oxidizing properties.

From a chemical point of view, the peroxyacids are peroxides of organic acids of general formula (I) 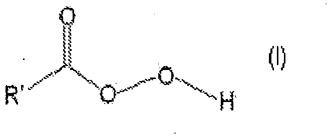 where R' is an alkyl or an aryl group.

In particular, the peroxyacetic acid, commonly referred to as peracetic acid, is the peroxyacid of the acetic acid, where R' is a methyl group (-CH₃).

Any consideration reported in the text of the present invention concerning the peracetic acid is to be extended to organic peroxyacids having overlapping chemical and physical properties and where R' is an alkyl or aryl group with up to 8 carbon atoms (e. g. perpropionic acid R'=CH₃-CH₂-, perbutanoic acid R' =- CH₂-CH₂-CH₃).

Peracetic acid is a liquid with an acrid odour. It is usually marketed in the form of aqueous solutions having concentrations of about 1.5%, 5%, 15% and 35% w/w. Owing to their properties of strong oxidants and broad-spectrum biocides, such solutions are used as bleaching, descaling agents, and especially as disinfectants and sterilizers in the medical-surgical field, typically for the disinfection of thermosensitive devices and equipments (e. g. endoscopes) and for environmental surfaces in operating rooms, etc..

The peracetic acid solutions, however, depending upon their concentration, are irritating or corrosive for the skin, noxious following ingestion, inhalation and contact with the skin, as well as combustive at very high concentrations, so that before use the concentrated solutions must be suitably diluted. Dilution, however, while on the one hand it solves the problem of hazard in handling and transport, on the other determines the hydrolysis of a predetermined amount of peracetic acid and the decomposition thereof in acetic acid, thereby reducing the available quantity of peracetic acid.

On an industrial scale, peracetic acid is prepared by reaction of hydrogen peroxide (commonly termed hydrogen peroxide solution) with acetic acid according to the following synthetic scheme: 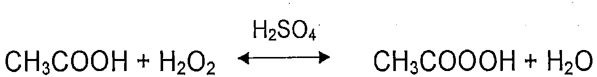 which leads to the formation of a mixture, at equilibrium, of reagents and products.

In order to promote rapid formation and stability of peracetic acid in solution, an acidic catalyst is added, e. g. a strong inorganic acid like, for example, sulphuric acid. It follows that the commercially available peracetic acid solutions are strongly acidic and, thus, notwithstanding their stability, they are particularly aggressive towards other materials to be treated.

Peracetic acid at concentrations ≥ 5% is corrosive and harmful following inhalation, ingestion and skin contact. This risk feature also affects the transport on the road, rail, by sea and air. In fact, for corrosive and combustive substances or preparations, and for organic peroxides, specific restrictions are provided as to their transport and also packaging and labelling.

Another preparation process for dilute solutions of peracetic acid, and therefore ready to use, comprises the reaction of *N,N,N*'*,N*'-Tetraacetylethylenediamine (TAED) or another powdered bleach activator (e. g. NOBS) with an alkaline adduct of hydrogen peroxide, such as sodium perborate orsodium percarbonate, in which the hydrogen peroxide reacts according to the following synthetic scheme:

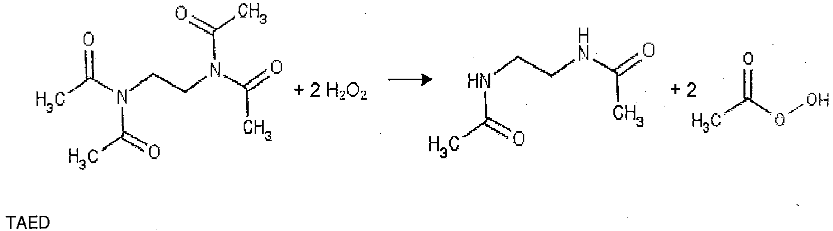

Such reaction occurs at a basic pH, which, besides allowing a rapid nucleophilic attack to the carbonyl carbon of the acetyl group with consequent rapid formation of peracetic acid, promotes the release of active oxygen with the consequent rapid decrease in the concentration of peracetic acid.

Furthermore, another drawback of this process is the presence of solid particles in suspension, which can clog the tiny channels of the endoscopes during the decontamination and/or disinfection of fiber optic apparatuses by using such solutions.

Other processes of preparation of peracetic acid solutions are described in the following patent documents.

U. S. patent application no. 2009/0043123 (Copenhafer) teaches a process for a rapid preparation and stabilization of peracetic acid diluted by adding acetic anhydride to an aqueous medium having a pH ranging from 5 to 12 and containing hydrogen peroxide and in which the molar ratio of hydrogen peroxide and acetic anhydride is greaterthan 1.

Such document shows that during the preparation of dilute aqueous peracetic acid, the pH increase in the aqueous solution results in the reaction rate being increased (see paragraph [0031]), and that in the preparation process certain features are used that are related to the pH and provide specific benefits, such as rapid production, stabilization and complete removal of residues of the dilute peracetic acid solutions (see paragraphs from [0045] to [0059]). As examples of pH regulator agents the common strong inorganic bases (alkaline and alkaline-earth hydroxides), or other inorganic buffer systems (phosphate, carbonate, etc.), and strong inorganic acids (sulphuric acid and others) are quoted.

The U. S. patent application no. 2005/109981 concerns a process and a system for extemporaneously generating decontaminating formulations.

So this document (see for example paragraph [0021]) teaches that the bleach activators must be stored separately from the water to avoid their hydrolysis, and hence the need for a system with three components, that is to say: Part A: containing water; Part B: containing hydrogen peroxide, and Part C: bleach activator. This three-component system, as it will be understood, is quite unpractical. Additionally, the reported organic bases ([0209] and claim 19) (triethanolamine TEA, N,N-dimethylethanolamine etc.) are tertiary amines provided with nucleophilic functional groups -OH (see structural formula of triethanolamine), and thus even if they are mixed with bleach activators in view of obtaining a two-component system, within a short time they are responsible for the attackto the acyl bonds of the donor molecules, especially the most electrophilic ones (such as N-acetylcaprolactam) with consequent loss or degradation of the active ingredient

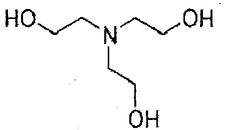

Owing to their lipophilic character, such amines are mixed in the lipophilic part A with the role of corrosion inhibitors. Their coexistence with the O-acetyl donor molecules (e. g. glycerol diacetate "diacetin" and propylene glycol diacetate and others) is limited in time, insomuch that considerations on the stability of such mixtures are not reported in the respective patent application.

The U. S. patent application no. 2005/109981 essentially discloses a decontamination aqueous solution comprising a nucleophilic agent having an oxidizing activity, a bleach activator, an inorganic base and water. The component that makes it possible to obtain a rapid reaction of dilute peracetic acid formation is the inorganic base, which is provided in the aqueous solution of hydrogen peroxide. The function of such base, chosen among the common inorganic bases (carbonate potassium, acetate potassium), is to raise the pH of the hydrogen peroxide solution, so that the high nucleophilic character of the hydro peroxide anion (H-O-O⁻), available at basic pH, can lead to the rapid formation of the organic peroxyacetic, when mixed with bleach activators. Without this inorganic base, the production of a decontamination solution based on peracetic acid would not take place, at least in times and at concentrations necessary to fulfil its function as a disinfectant and sterilizer. As it will be understood, such inorganic base, once added to the aqueous part B, is a source of destabilization for the aqueous hydrogen peroxide, which for full stability thereof must be maintained at an acidic pH or converted to solid form as percarbonate or perborate, or be adsorbed on a solid support (e. g. amorphous silica, etc..), or this third element must be added to the oxygenated water immediately before adding the lipophilic part A, containing the bleach activator, as it is also disclosed in the U. S. patent application no. 2009/0043123.

The patent EP-0 953 283 B9 teaches, instead, the extemporaneous preparation of a peracetic acid solution by mixing a parent aqueous solution comprising *O*-acetylated or N-acetylated compounds with a hydrogen peroxide-based *Activator.* As stated above, the aqueous environment in which the donorcompounds of O-acetylated N-acetylated acetyls are dispersed inevitably makes them unstable, and thus they quickly undergo hydrolysis. Consequently, the teaching of this patent does not find practical application, and does not make it possible to have a stable system up to at least 2 years and suitable for preparing extemporaneously diluted and concentrated peracetic acid.

The patent EP-1 077 606 B1 describes the preparation of an antimicrobial composition and its use for the disinfection of surfaces and/or surgical instruments, endoscopes, circuits, etc. by mixing an oxygen generating liquid base, such as hydrogen peroxide, and an acetyl group liquid generator constituted by N-acetylcaprolactam. To speed up the perhydrolysis reaction, the solution of hydrogen peroxide is maintained at neutral-basic pH by adding a nucleophilic and basic compound, such as sodium or potassium hydroxide or an alcoholamine (e. g. monoethanolamine, diethanolamine, triethanolamine), or a basic adduct of hydrogen peroxide (e. g. sodium perborate or percarbonate), which could not be previously mixed with the N-acetylcaprolactam since it would become degraded. In these conditions, however, hydrogen peroxide is very unstable, as it undergoes dismutation into water and oxygen, and the latter escapes from the solution in gaseous form, thus reducing the concentration and simultaneously lowering the pH value of the solution itself. The release of gaseous oxygen also poses problems from a practical point of view, since it is necessary to store and transport the oxygen-donating solution in cold ambient and in containers with a vented cap in order to prevent excessive swelling of the packaging.

The patent EP-1 371 643 discloses, instead, an extemporaneous preparation process by mixing an activator with a source (or generator) of peroxide. In it the same considerations are made as those to be found in U.S. patent application no. 2005/109981 in connection with the absence of water and the nucleophilic character of all the ingredients (solvents, emulsifiers and dispersants) mixed in the liquid phase with the molecules that release acetyl groups.

It is then felt the need of having a system with only two liquid components, preserved separately and stable overtime (at least 2 years), and once mixed they can provide extemporaneously, at the place of use, an organic peroxyacid based aqueous or hydro alcoholic solution, in particular diluted and/or concentrated peracetic acid. This system is not only stable over time, but is also no longer dangerous for human health, safety, environment and transport, compared to a solution of organic peroxyacid (peracetic acid) that is formed *in situ* by mixing two liquid components.

The main object of the present invention is to provide a system of two liquid components that are stable over time (at least 2 years) and that once mixed together give rise extemporaneously to a solution of organic peroxyacid (peracetic acid) having a suitable stability for being used as a biocide, disinfectant, sterilizer, bleach, descaler and detoxifier.

Another object of the present invention is to provide said two liquid component system, with a respective preparation process of a concentrated or diluted solution of peracetic acid, easy and safe to handle and transport.

A further object of the present invention is to provide said system, wherein the chemical composition of its two liquid components is such as to enable a rapid (within a few minutes or seconds) *in situ* preparation of a peracetic acid solution within a broad concentration range.

A further object of the present invention is to provide said system and preparation, that make it possible to obtain a disposable or reusable peracetic acid solution which is stable for the time required by a specific use.

A further object of the present invention is to provide a solution of peracetic acid which is stable and ready for direct use as an antimicrobial, in processes of disinfection and/or chemical, manual sterilization, i. e. through immersion in basin, and automated sterilization, by recirculation in washing machines for endoscopes or surgical instruments or even thermosensitive instrumentation, and/or environmental surfaces, especially in the medical-surgical field.

Further examples of suitable application of this system and preparation include disinfection of equipment for food processing, disinfection of areas for the breeding of animals, getting rid of moulds in storage warehouses, bleaching of textiles and garments, descaling and simultaneous disinfection of internal circuits of hemodialysis generators, of distribution systems of food and beverages (CIP), decontamination from chemical and biological warfare (anthrax and plague) and toxic industrial chemicals (cyanide and phosgene), and others where a whitening, oxidizing, descaling, disinfecting and sterilizing effect is required.

According to a first aspect of the present invention, a system is provided of two liquid components which are separately and stably kept for a long time, and designed to produce extemporaneously, when mixed together, an aqueous or hydroalcoholic solution of a concentrated or dilute organic peroxyacid, the system including a lipophilic component and a hydrophilic component, with the lipophilic component including at least one donor of at least one acetyl group, whereas the hydrophilic component includes an aqueous or hydroalcoholic solution at acidic pH containing an oxygen donor, the lipophilic component comprising at least one pH regulator/raiser including at least one aliphatic amine with weak or no nucleophilic character and free from nucleophilic functional groups, the aliphatic amine acting as pH raiser/ regulator at the time of mixing together the two hydrophilic and lipophilic components, thereby making it possible to obtain a fast and efficient production of peracetic acid.

According to another aspect of the present invention, there is provided a process for extemporaneously preparing a solution of a freshly organic peroxyacid, comprising the following steps:
- providing a system according to the present invention; and
- mixing together the two components of this system in pre-measured quantities.

Advantageously, the process comprises the following steps:
- providing a lipophilic component including a donor of at least one acetyl group and at least one pH regulator including at least one aliphatic amine with weak or no nucleophilic character and free from functional nucleophilic groups;
- providing a hydrophilic component including an aqueous or hydroalcoholic solution at acidic pH, or at a pH below 5.5, containing an oxygen donor,
- separately keeping the lipophilic and hydrophilic components; and afterwards
- mixing together the lipophilic and hydrophilic components thereby determining the following steps:
- removal by the at least one aliphatic amine with weak or no nucleophilic character of hydrogen ions H⁺ or H₃O⁺ from the hydrophilic component, whereby quickly raising the pH while increasing the nucleophilic character of that oxygen donor;
- quick attack by the oxygen donor to the carbonyl carbon of the acetyl group donor with consequent rapid formation of an organic peroxyacid;
- lowering by the organic peroxyacid of pH to the acidic range, thereby obtaining a substantially stable solution of organic peroxyacid in combination with an amine/ammonium ion system.

The present invention concerns therefore mainly a system of two liquid components, a lipophilic and a hydrophilic component.

The liquid lipophilic component, the subject matter of the present invention, comprises one or more donors or donor molecules of one or more acetyl groups and one or more pH regulators.

Advantageously, the donor/donors of one or more acetyl groups is/are a/some molecule/molecules chosen in the group comprising N-acetylated and *O*-acetylated compounds.

More particularly, the currently preferred N-acetylated compounds comprise *N,N,N*'*,N*'-tetraacetylethylenediamine, tetraacetylglycoluril, one or more (C₄-C₁₀) N-acetyllactams, preferably N-acetylcaprolactam, and one or more N-acetylimide having a number of carbon atoms greater than or equal to 4, such as N-acetylsuccinimide, N-acetylphthalimide and 2-n-nonyl-N-acetylsuccinimide, whereas the currently preferred *O*-acetylated compounds comprise acetic anhydride, acetylsalicylic acid and acetate esters of sugars such as pentaacetate glucose, octaacetate sucrose, esaacetate mannitol, and polyols such as glycerol diacetate or propylene glycol diacetate.

The use of N-acetylated or *O*-acetylated compounds as activators of peroxide groups has already been proposed, e. g. as disclosed in the patent documents WO-95/20876, US-4 128 494 and US-5 654 269, in which compounds having the following general formula are described: 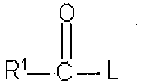 where R¹ is a methyl and L is a good leaving group with a nitrogen or oxygen atom directly linked to the carbonyl carbon.

As is known, in fact, an N-acetylated or *O*-acetylated group has electrophilic chemical properties, which make it susceptible to an attack by a nucleophilic, i. e. a chemical species suitable for donating an unshared electronic doublet, with subsequent rupture, respectively, of the amide or ester bond and the release of the acetyl group from the donor molecule.

Examples of molecules and ions that behave as nucleophilic are water (H₂O), ammonia (NH₃), alcohols (ROH), ethers (ROR), the primary (RNH₂), secondary (R₂NH) and tertiary (R₃N) amines, the hydroxyl ion (OH-), the alcoholate (RO-) and thiolate (RS⁻) ions. The nucleophilic character of these species depends on several factors, among which the most important are the pKₐ value (negative logarithm of the acidic dissociation constant of a protonated chemical species), the electronegativity, the presence in the chemical species of electron-donor or electron-attractor groups or atoms, the steric hindrance and the nature of the solvent. In so far as the steric hindrance is concerned, the presence in a molecule or ion of bulky substituents, so-called 'cumbersome' substitutes, has a negative effect on its nucleophilic character, and thus reduces its tendency to react with an electrophilic species.

Factors such as electronegativity and the presence of an active function with an electron-attractor or electron-donor effect affects the electrophilic properties of the N-acetyl or O-acetyl group, in the sense that such groups are the more reactive, and thus electrophilic the more their carbonyl carbon is affected by the electron-attraction effect exerted by carbonyl groups present in a vicinal position thereto.

In order to avoid the preventive release of the acetyl group by the donor molecules, the pH regulator provided in liquid mixture with such molecules, besides having lipophilic and alkalizing properties, must have a weak or no nucleophilic character.

Such pH regulator includes at least one aliphatic amine with weak or no nucleophilic character and free from nucleophilic functional groups.

The expression "weak or no nucleophilic character" in this description and in the claims means no or almost no reactivity of a nucleophilic compound to the carbonyl carbon of the acetyl group.

Owing to these properties, the pH regulator does not degrade over time the donor molecule of acetyl groups with which it is combined and upon being mixed with the other liquid hydrophilic component of the present invention, oxygen-base active at acidic pH, rises rapidly the pH of the mixture and increases at the same time the nucleophilic character of the oxygen donor of the hydrophilic component, by removing hydrogen ions (H⁺ or H_{3O}⁺), so that the peroxide or hydro peroxide anions, in greater amount at a neutral basic pH (see acidic dissociation equilibrium of hydrogen peroxide), 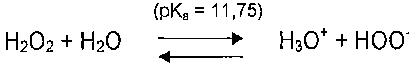 by being much more reactive, can attack the carbonyl carbon of the donor of acetyl group, leading quickly to the formation of peracetic acid, according to the reaction scheme below.

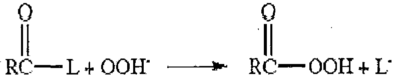

Subsequently, the peracetic acid thus formed causes the pH to lower down to the acidic interval, thus obtaining a substantially stable solution of peracetic acid in combination with an amine/ammonium ion system, the latter being formed upon mixing and having, as it will be also said hereinafter, a synergistic biocidal activity with the organic peroxyacid.

pH regulators or alkalizing agents of lipophilic nature and with weak nucleophilic are chosen from the group comprising one or more cyclic amines of Formula (II): 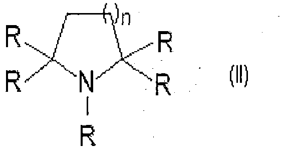 in which
R is hydrogen and/or a methyl and/or an alkyl group, and
n is 0, 1 or 2;
and one or more tertiary amines of Formula (III) 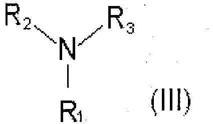 in which R₁, R₂ and R₃ are an alkyl group.

The term "alkyl" used here means a linear or branched alkyl group containing two or more carbon atoms. Examples of such alkyl group include: ethyl, n-propyl, isopropyl, n-butyl, isobutyl, sec-butyl, tert-butyl, n-pentyl, isopentyl, and neopentyl.

Advantageously, amines of Formula (II) include 2,2,6,6-tetramethylpiperidine, 2,2,5,5-tetramethylpyrrolidine, 2,6-dimethylpiperidine, 2,5-dimethylpyrrolidine, N-ethylpyrrolidine and *N*-ethylpiperidine, and preferably 2,2,6,6-tetramethylpiperidine, while the amine group of Formula (III) advantageously comprises *N*,*N*,*N*-diisopropylethylamine and N, *N*,*N*-triethylamine.

To prepare an extemporaneous solution, preferably in a few minutes or seconds, of peracetic acid according to the method of the present invention, the concentration percentage (% w/w) of the aliphatic amine to be used in the liquid lipophilic component is a function of the quantity relationship between the two liquid components of the system, the subject matter of the present invention, the acidity degree of the liquid hydrophilic component acting as an oxygen generator (hydrogen peroxide in an aqueous solution can have different acidity degrees as a function of its concentration and the applied stabilization), and the value of basic neutral pH to be reached in the reaction mixture, which value in turn controls the rate of formation of peracetic acid or peracetic ion. Depending upon these factors, its concentration in the liquid lipophilic component ranges anyway from 0.1% w/w to 10.0% w/w.Such composed system, compared to the state of the art, has the advantage of being stable over time, even under unfavourable temperature conditions. At room temperature (about 25°C), for example, a long-time stability is ensured, preferably of at least two years.

Moreover, the two liquid component system according to the present invention as described above, owing to its qualitative and quantitative composition, is not more dangerous for human health, the collective safety and the environment, then the mixtures obtained according to the process provided for in the present invention, so that it can be handled and transported by air or by sea or by land without requiring, precautions or preventive measures, or requiring them at a reduced rate.

The two liquid component system according to the present invention is directed to the instant preparation of an aqueous or hydro alcoholic solution of a peroxyacid, in particular, concentrated or diluted, disposable or reusable peracetic acid, which is stable throughout the time required for exerting its function as disinfectant, sterilizer, bleach, descaler and detoxifier in many fields (e.g. health, agricultural and food, zootechnical, professional, industrial, textile and others). Such preparation is carried out at moment of use according to the process of the present invention which substantially comprises to cause the ingredients of two separately stored liquid components to react by mixing them.

Advantageously, the liquid hydrophilic component, according to the present invention includes an aqueous acidic solution (pH < 5.50 pH units) of hydrogen peroxide at a concentration by weight between 0.01% and 90%, and preferably between 3% and 35%.

Under these conditions, the aqueous solution of hydrogen peroxide is stable and can be stored for at least two years at room temperature (about 25°C) and for 14 days at 54°C without undergoing chemical and physical changes, as determined by the CIPAC method "MT 46 Accelerated Storage Tests by Heating".

The mixing of the aqueous acidic solution of hydrogen peroxide with the liquid lipophilic component described in the present invention, owing to the presence of a specific pH regulator (aliphatic amine, preferably tertiary amine, with a low nucleophilic character and with no nucleophilic functional groups), determines a sudden pH increase in the reaction mixture at basic neutral values, e. g. in the range from 7.0 to 12.5 pH units. This increase allows the production reaction of peracetic acid to proceed quickly and with high yields %. As can be seen by comparing the compositions and graphs in the Examples 3 and 4, the absence of a specific pH regulator in the lipophilic component, and its replacement with a solvent having a low nucleophilic character (isopropyl alcohol), but with no effect on pH, as taught by the patent EP-1 371 643, other conditions being equal, does not lead to any formation of peracetic acid.

This proofs the fundamental importance of this ingredient in the system and its respective process, both the subject matter of the present invention.

In the aliphatic tertiary amines with low or null nucleophilic character, since the nitrogen atom is surrounded by bulky alkyl groups, only the proton H⁺ is small enough to enter therein. In this way, such amines act as subtractors of protons or rather as "proton scavengers".

The pH regulator (at least one aliphatic amine, preferably a tertiary one with weak or no nucleophilic character and void of nucleophilic functional groups) in accordance with the present invention is the only one, compared to those described in earlier documents mentioned above, that can be maintained permanently in combination with molecules releasing acetyl groups. Indeed, in addition to the low or no nucleophilic character, at room temperature it is liquid and, owing to its lipophilic nature, blends perfectly well with the liquid molecules releasing acetyl groups which are also lipophilic.

The attempt to match the pH adjusters mentioned in the prior art patent documents, such as alkaline or alkaline-earth hydroxides and amino alcohols (triethanolamine, 2-amino-2-methylpropanol - AMP) results in a rapid degradation of the acetyl group-releasing molecule with the formation of acetic-acetate acidic system that buffers the pH of the mixture at acidic values, that are no longer suitable for a rapid formation of peracetic acid.

In the system and process provided in the present invention, however, the quality and quantity of aliphatic tertiary amine is such that after the initial sharp pH rise in the reaction mixture, the peracetic acid, being formed in progressively larger quantities, rapidly lowers the pH to acidic values, preferably between 3.0 and 5.5 pH units, which gives a stable peracetic acid solution.

The reaction according to the process of the present invention can take place at any temperature between 0°C and 80°C, preferably between 10°C and 60°C.

The process according to the present invention allows the preparation of peracetic acid solutions at any concentration between 0.00001 % and 40% w/w (0.1 - 400000 ppm) or % w/v, preferably between 0.01 % and 35% w/w or w/v (100 - 350000 ppm). More specifically, by varying the concentration of hydrogen peroxide used in the process according to the present invention, and its molar ratio with the acetyl groups donor, various solutions of peracetic acid both diluted and concentrated can be obtained.

Dilute solutions of peracetic acid have a concentration by weight preferably between 0.0001 % and 0.30%, while concentrated solutions have a concentration by weight preferably between 1.5% and 35%.

In general, the concentrated solutions of peracetic acid are very corrosive, so that their transport as well as their handling are subject to specific restrictions imposed by law. With the system and process according to the present invention, such solutions can be prepared only at the time of use, and after proper dilution they can be used as liquid disinfectants or sterilizers or for other purposes, thereby overcoming the above-mentioned problems.

After said reaction stage, the process according to the present invention therefore provides for the solution being diluted by adding a specified quantity of water until the desired concentration for the specific intended application is reached.

Advantageously, to the hydrophilic or lipophilic component of this system one or more chemical additives selected from alcohols such as ethanol and isopropyl alcohol, surfactants, dispersants, colorants, anticorrosion agents, diols and polyols can be added.

Such chemical additive/additives, if added to the lipophilic component according to the present invention, as already disclosed in the patent EP-1 371 643, must be anhydrous and with no nucleophilic character in order to prevent degradation of the acetyl group donor.

Solutions of peracetic acid, prepared extemporaneously according to the process of the present invention, can be either aqueous or hydro alcoholic solutions, in order to take advantage of the well-known synergistic biocide effect between alcohols and peracetic acid. Moreover, such solutions, prepared according to the process of the present invention, can be used in disinfection processes and cold chemical sterilization of medical-surgical instruments and/or equipment, filtration systems and environmental surfaces. Medical-surgical instruments and/or equipment include in particular thermosensitive fiber optic devices, such as rigid or flexible endoscopes and transesophageal probes that due to their thermosensitivity cannot be sterilized by being heated.

Furthermore, the extemporaneous solutions of peracetic acid, prepared according to the teachings of the present invention, can be prepared, diluted and used directly in automatic washing machines, e.g. for endoscopes, surgical instruments and bedpans, or for descaling hemodialysis generators.

The chemical N-acetylated and *O*-acetylated compounds, the amines of Formula (II) and (III), the oxygen generator (hydrogen peroxide) and the additive(s) of the present invention are all commercially available materials, that can be purchased e. g. from Sigma-Aldrich Inc., or BASF Italia S.r.l..

Some examples of the composition of the two liquid component (hydrophilic + lipophilic) system, of the preparation process of an aqueous or hydroalcoholic solution of peracetic acid (concentrated or diluted) and their direct - or after dilution - practical use as disinfectants or cold chemical sterilizers of thermosensitive medical-surgical instruments (endoscopes), as bleaches of textiles and garments, as disinfectants and descaler of the CIP plants, and for the decontamination of sites for housing animals, of work surfaces in food industry and other typical applications of peracetic acid solutions are given hereinbelow.

### Example 1

### Extemporaneous preparation of a dilute solution (or ready to use) of peracetic acid.

A dilute solution of peracetic acid was prepared according to the following reaction scheme (Reaction scheme 1) and the conditions listed in the following table (Table 1).

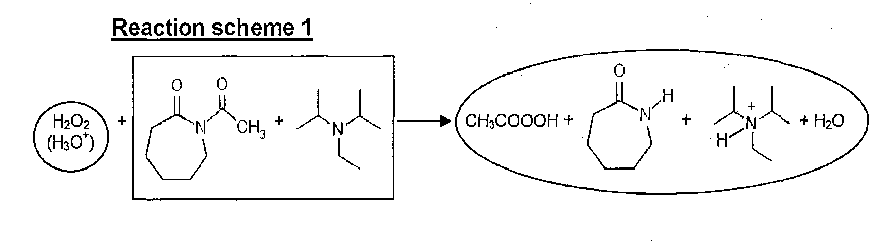

**Table 1**

| **Ingredient** | % **w/w** | **Chemical-physical properties** | **Quantity (g)** |
|---|---|---|---|
| **A: HYDROPHILIC component - Oxygen generator** | | | |
| Hydrogen peroxide | 3.00 | pH = 3.80 - 4.20 pH units at 20°C | 1000 |
| Distilled water q.s. at | 100.00 | | |

| **LIPOPHILIC component - Donor of an acetyl group and tertiary amine** | | | |
|---|---|---|---|
| *N*-acetylcaprolactam | 96.875 | | 6.4 |
| *N,N,N-*diisopropylethylamine | 3.125 | | |
| Products | | | |

| **Peracetic acid sotution + system amine/ammonium ion** | | | |
|---|---|---|---|
| Hydrogen peroxide | 2.91 | pH = 7.80 (T_{0 minutes}) - 6.33 (T_{5 minutes}) - 4.85 (T_{30 minutes}) - 3.75 (T_{120 minutes}) - 3.70 (T_{14 days}) | 1006.4 |
| Peracetic acid | 0.15-0.09 | | |
| Caprolactam | 0.46 | | |
| System amine/ammonium ion | 0.02 | Stability: > 14 days at 25 °C for concentrations > 0.09% w/v | |
| Distilled water q.s. at | 100.00 | | |

The hydrophilic component A, i. e. an aqueous solution of hydrogen peroxide, was prepared at 3% by weight by diluting 85.71 g of hydrogen peroxide at 35% w/w with distilled water *as needed* at 1000 g and the pH was determined at 20°C. 6.4 g of the lipophilic component B were prepared separately, i. e. the mixture containing N-acetylcaprolactam (6.20 g) and *N*,*N*,*N*-diisopropylethylamine (0.20 g) and such component was then added to 1 kg of the hydrophilic component A, i. e. of the hydrogen peroxide solution at 3% w/w in order to obtain instantly (after 20 seconds) a solution of peracetic acid having a concentration by weight between 0.09% and 0.15% (900 - 1500 ppm), hydrogen peroxide (oxygenated water) in excess (2.9% w/w) and the amine/ammonium ion system in an amount of 0.02 w/w.

Both the pH variation of the peracetic acid solution thus obtained and the concentration variation over time of peracetic acid were determined, and the results are reported in Table 1 and in the following diagrams A and B.

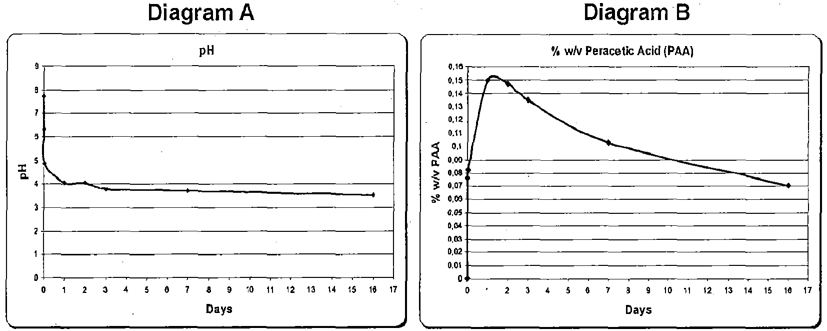

From diagram A one can see how, at the time of mixing the two components (hydrophilic and lipophilic) (t = 0), the pH of the hydrogen peroxide solution (component A: hydrophilic), maintained at acidic pH, instantly took a neutral basic value (7.0 - 8.0), which allowed the formed hydroperoxide anions (HOO⁻) to effectively lead a nucleophilic attack to the carbonyl carbon of the acetyl group, thus making the production of peracetic acid or peracetate anion fast and sudden. After this initial instant, the rapid formation of peracetic acid (as can be seen from diagram B) is accompanied by an equally rapid decrease in pH until the acidic values, within the range of 3.5 to 5.5, which allows the solution to preserve its stability over time.

The pH value obtained is not such as to consider the solution as corrosive. Indeed, this value is compatible with the skin (approximately pH = 5), so that the solution has excellent compatibility also with various materials to which it is applied.

The solution as such was tested for biocidal activity (bactericidal, fungicidal, mycobactericidal, tuberculocidal, virucidal and sporicidal), according to the current European standards and in accordance with the CEN methods TC/ 216, in orderto show its validity and therefore its use as a high-level disinfectant and/or cold chemical sterilizer of medical-surgical instruments even of the thermosensitive ones, such as fiber optic equipment (endoscopes, bronchoscopes, fiberscopes and others). The following table shows, for each adopted standard, the results of such tests in terms of contact time.

| **Activity** | **European Standard** | **Phase/step** | **Time** |
|---|---|---|---|
| Bactericidal | EN 13727 | 2,1 | 1 minute |
| | EN 14561 | 2,2 | 1 minute |
| Fungicidal | EN 13624 | 2,1 | 5 minutes |
| | EN 14562 | 2,2 | 5 minutes |
| Mycobactericidal and Tuberculocidal | EN 14348 | 2,1 | 5 minutes |
| | EN 14563 | 2,2 | 5 minutes |
| Virucidal | EN 14476 | 2,1 | 5 minutes |
| Sporicidal | AFNOR NFT 72-300 | 2,1 | 10 minutes |
| | AFNOR NFT 72-190 | 2,2 | 10 minutes |

Furthermore, in order to show the synergistic biocide effect between the amine/ammonium ion system and peracetic acid, a comparative test of sporicidal activity was made, between the solution obtained from the system and process described above, with an aqueous solution having the same peracetic acid concentration and obtained by diluting with distilled water a solution of a commercially available concentrated solution of peracetic acid (peracetic acid - APA 5% w/w) and with an aqueous solution based on the amine/ammonium ion system and distilled water in the same proportions in which they are present in the solution. The results of this comparison are listed below.

| **European Standard** | **C: Peracetic acid solution 0,10% + system amine/ ammonium ion 0,02% + hydrogen peroxide (HP) 2,91%** | **Aqueous solution system amine/ ammonium ion 0,02% + HP 2,91%** | **Peracetic acid solution 0,10% (20 g of APA 5% brought to 1000 ml l with water)** + **HP 2,91%** |
|---|---|---|---|
| AFNOR NF T 72-190 | 10 minutes | N. A. (no activity) | 20 minutes |

As shown in above table, the system amine/ammonium ion in combination with hydrogen peroxide at 2.91% has not in itself sporicidal activity. However, owing to its presence in an aqueous solution of peracetic acid at 0.1 % (1000 ppm), the time required for the completion of sporicidal activity is halved. This indicates that the system amine/ ammonium ion promotes or accelerates the sporicidal activity typical of the peracetic acid with a synergistic effect.

### Example 1a

### Extemporaneous preparation of a diluted (or ready for use) hydroalcoholic solution of peracetic acid.

A diluted hydroalcoholic solution of peracetic acid was prepared according to the following reaction scheme (Reaction Scheme 1a) and the conditions are shown in the following table (Table 1a).

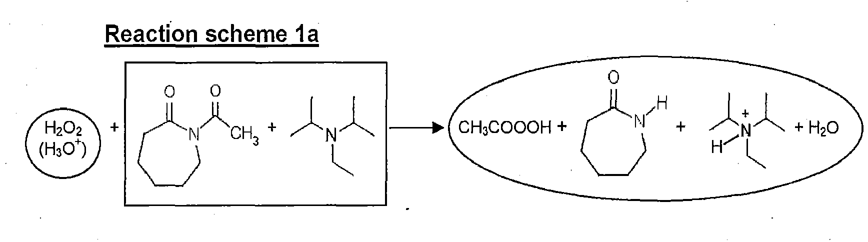

**Table 1a**

| **Ingredient** | **%w/w** | **Chemical-physical properties** | **Quantity (g)** |
|---|---|---|---|
| **A: HYDROPHILIC component-Oxygen generator** | | | |
| Hydrogen peroxide | 3.00 | pH = 3.90 - 4.30 pH Units at 20°C | 1000 |
| Isopropylic alcohol | 20.00 | | |
| Distilled water q.s. at | 100.00 | | |

| **UPOPHILIC component - Donor of an acetyl yl group and tertiary amine** | | | |
|---|---|---|---|
| *N*-acetylcaprolactam | 96.875 | | 6.4 |
| *N,N,N*-diisopropylethylamine | 3.125 | | |

| **Peracetic acid solution + system amine/ammonium ion** | | | |
|---|---|---|---|
| Hydrogen peroxide | 2.91 | pH = 7.80 (T_{0 minutes}) - 6.33(T_{5 minutes}) - 4.85 (T_{30 minutes}) - 3.75 (T_{120 minutes}) - 3.70 (_{T14 days}) | 1006.4 |
| Peracetic acid | 0.15-0.09 | | |
| Isopropylic alcohol | 19,87 | | |
| Caprolactam | 0.46 | | |
| System amine/ammonium ion | 0.02 | Stability: > 14 days at 25 °C for concentrations > 0.09% w/v | |
| Distilled water q.s. at | 100.00 | | |

The hydrophilic component, i. e. a hydroalcoholic solution of hydrogen peroxide at 3% by weight, was prepared by diluting 85.71 g of hydrogen peroxide at 35% w/w with distilled water as needed to 800 g, by adding, after thorough mixing, the remaining 200 g of isopropyl alcohol, and the pH was determined at 20°C. Separately, 6.4 g of the lipophilic component were prepared, i. e. a mixture containing N-acetilcaprolactam (6.20 g) and N, N, N-diisopropylethylamine (0.20 g), and the mixture thus obtained was added to 1 kg of hydroalcoholic solution of hydrogen peroxide at 3% w/w in orderto obtain instantly (after 15 seconds) a hydroalcoholic solution of peracetic acid having a concentration by weight between 0.09% and 0.15% (900 - 1500 ppm), hydrogen peroxide (oxygenated water) in excess (2.91 % w/w), system amine/ammonium ion in the amount of 0.02% w/w and isopropyl alcohol 19.87% w/w.

Both the variation in pH of the solution thus obtained and the variation of the concentration in time of peracetic acid were determined, obtaining similar results even slightly higher than the aqueous solution itself; the results are shown in Table 1 a and in the following Diagrams A1 and B1.

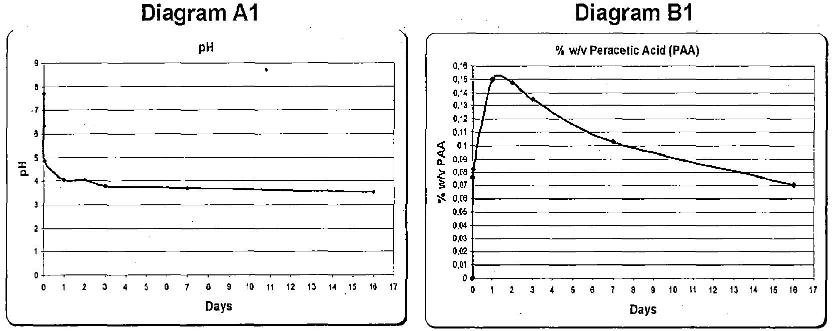

From diagram A1 one can see that, at the time of mixing the two components (hydrophilic and lipophilic) (t = 0), the pH of the hydrogen peroxide solution (component A: hydrophilic) kept at an acidic pH, instantly took a neutral basic value (7.0 - 8.0), which allows the formed hydroperoxide anions (HOO-) to effectively bring a nucleophilic attack to the carbonyl carbon of acetyl group which makes the production of peracetic acid or peracetic anion fast and sudden. After this initial instant, the rapid formation of peracetic acid (as shown in diagram B1) is accompanied by a rapid pH lowering, which, by reaching acid values within the range of 3.5 - 5.5, allows the solution to preserve its stability over time.

The solution as such was tested for biocidal activity (bactericidal, fungicidal, mycobactericidal, tuberculocidal, virucidal and sporicidal), according to the current European standards and in accordance with the methods CEN TC/ 216, in order to show its validity as a high-level disinfectant and/or cold chemical sterilizer of medical-surgical instruments even thermosensitive ones, such as thermosensitive fiber-optic equipment (endoscopes, bronchoscopes, fiberscopes and others). The table below shows for each standard adopted the results of these tests in terms of contact time.

| **Activity** | **European Standard** | **Phase/step** | **Time** |
|---|---|---|---|
| Bactericidal | EN 13727 | 2,1 | 1 minute |
| | EN 14561 | 2,2 | 1 minute |
| Fungicidal I | EN 13624 | 2,1 | 1 minute |
| | EN 14562 | 2,2 | 1 minute |
| Mycobactericidal and Tuberculocidal | EN 14348 | 2,1 | 5 minutes |
| | EN 14563 | 2,2 | 5 minutes |
| Virucidal | EN 14476 | 2,1 | 5 minutes |
| Sporicidal | AFNOR NFT 72-300 | 2,1 | 5 minutes |
| | AFNOR NF T 72-190 | 2,2 | 10 minutes |

When comparing these results with those of the aqueous solution of the example 1, one will note the synergistic effect exerted by the isopropyl alcohol in so far as the fungicidal activity is concerned in both steps (i.e. Phase 2/Step 1 = suspended and Phase 2/Step 2 on a support or surface) and the sporicidal activity in suspension (Phase 2/Step 1).

### Example 2

### Extemporaneous preparation of a concentrated solution of peracetic acid (to be diluted before use).

A concentrated solution of peracetic acid was prepared according to the following reaction scheme (Reaction scheme 2) and the conditions listed in the following table (Table 2).

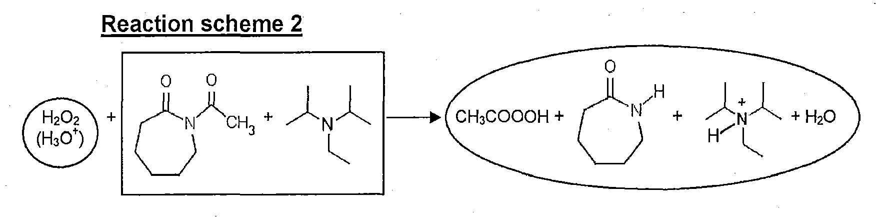

**Table 2**

| **Ingredient** | **% w/w** | **Chemical-physical properties** | **Quantity (g)** |
|---|---|---|---|
| **A: HYDROPHILIC component - Oxygen generator** | | | |
| Hydrogen peroxide | 30.00 | pH = 2.30 - 2.40 pH units at 20°C | 44.8 |
| Distilled water q.s. at | 100.00 | | |

| **LIPOPHILIC component - Donor of an acetyl group and tertiary amine** | | | |
|---|---|---|---|
| *N*-acetylcaprolactam | 92.40 | | 23.0 |
| *N,N,N*-diisopropylethylamine | 7.60 | | |
| Products | | | |

| **Peracetic acid solution + system amine/ammonium ion** | | | |
|---|---|---|---|
| Hydrogen peroxide | 13.5 - 14.5 | pH = 7.60 (T_{0 minutes}) - 6.40 (T₅ minutes) - 6.20 (T₁₀ minutes) - 4.85 (T_{30 minutes}) - 3.75 (T₁₂₀ minutes) | 67.8 |
| Peracetic acid | > 10.0 | | |
| Caprolactam | 25.28 | | |
| System amine/ammonium ion | 2.58 | | |
| Distilled water q.s. at | 100.00 | | |

A solution of hydrogen peroxide was prepared at 30% w/w by diluting 38.4 g of hydrogen peroxide at 35% w/w with distilled water as needed at 44.8 g and the pH was determined at 20 °C. A mixture of 23.0 g was prepared separately containing21.25 g of N-acetylcaprolactam and 1.75 g of *N*,*N*,*N*-diisopropylethylamine and was then added under agitation to the hydrogen peroxide solution in order to obtain immediately a concentrated solution of peracetic acid having a concentration > 10.0 % w/w after 3 minutes. During the reaction, an increase in temperature of about 15°C was observed.

Both the pH variation over time of the peracetic acid solution and of the percentage concentration weight/ volume of peracetic acid thus obtained were determined, and the results are reported in Table 2 and in the following diagrams C and D.

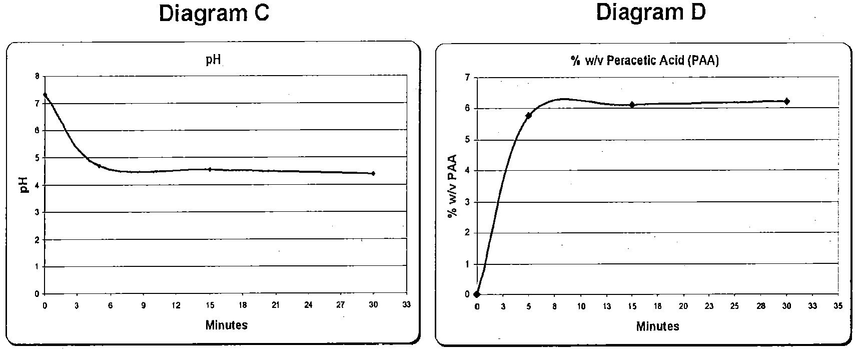

In diagram C one can note that, at the time of mixing the two components (t = 0), the acidic pH of the hydrogen peroxide solution instantly took a neutral basic value (7.0 - 8.0), which allows the hydroperoxide anions (HOO⁻) formed to effectively lead a nucleophilic attack to the carbonyl carbon of the acetyl group. Without this instantaneous increase in pH the perhydrolysis reaction does not take place (as one can see in Example 4).

After the initial instant, the rapid formation of peracetic acid (as shown in diagram D) is accompanied by an equally rapid decrease in pH down to acidic values, within the range of 4.0 - 5.0, which makes it possible for the solution to preserve its stability.

Such solution was diluted with deionised water at 1.5%, 2.0% and 2.5% v/v in order to evaluate the sporicidal activity according to the current European standard EN 14347 (Phase 1), of 3 possible dilutions of use. Indeed, such a concentrated aqueous solution of peracetic acid could be obtained according to the teachings of the present invention by mixing the two components in a suitably prepared reaction chamber in a washing machine and/or disinfecting machine for endoscopes, and then automatically diluting it with filtered water in the sterilization basin of said machine according to the dosage that provides the best chances for sporicidal activity. Thus, for all 3 dilutions referred to above, the solution was fully effective without detecting any growth (cfu / ml = 0) in all the incubated plates.

### Example 2a

### Extemporaneous preparation of a concentrated aqueous solution of peracetic acid (to be diluted before use).

A concentrated aqueous solution of peracetic acid was prepared according to the same reaction scheme and the same conditions as described in Example 2, the only variant being the replacement of the aliphatic tertiary amine with another amine having the same characteristics (Table 2a).

**Table 2a**

| **Ingredient** | **% w/w** | **Chemical-physical properties** | **Quantity (g)** |
|---|---|---|---|
| **A: HYDROPHILIC component - Oxygen generator** | | | |
| Hydrogen peroxide | 30.00 | pH = 2.30 - 2.40 U di pH a 20°C | 44.8 |
| Distilled water q. s. at | 100.00 | | |

| **LIPOPHILIC component - Donor of an acetyl group and tertiary amine** | | | |
|---|---|---|---|
| *N*-acetylcaprolactam | 93.00 | | 23.0 |
| *N*,*N*,*N-triethylamine* | 7.00 | | |

| **Peracetic acid solution + system amine/ ammonium ion** | | | |
|---|---|---|---|
| Hydrogen peroxide | 13.5 - 14.5 | pH = 7.60 (T_{0 minutes}) - 6.40 (T₅ minutes - 6.20 (T_{10 minutes}) - 4.85 (T_{30 minutes}) - 3.75 (T₁₂₀ minutes) | 67.8 |
| Peracetic acid | > 10.0 | | |
| Caprolactam | 25.28 | | |
| System amine/ammonium ion | 2.58 | | |
| Distilled water q. s. at | 100.00 | | |

A solution of hydrogen peroxide was prepared at 30% w/w by diluting 38.4 g of hydrogen peroxide at 35% w/w with distilled water as *needed* at 44.8 g and the pH at 20°C was determined. Separately, 23.0 g were prepared of a mixture containing 21.39 g N-acetylcaprolactam and 1.61 g of *N,N,N*-triethylamine and the latter was added under agitation to the hydrogen peroxide solution to obtain immediately a concentrated solution of peracetic acid having a concentration >10.0% w/w after 3 minutes. During the reaction, an increase in temperature of about 15°C approx was observed.

Both the variation in time of pH and the percentage concentration weight/volume of peracetic acid in the solution thus obtained were determined, and the same results as reported in Example 2 were obtained.

### Example 3

### Extemporaneous preparation of a concentrated solution of peracetic acid (to be diluted before use).

A concentrated solution of peracetic acid was prepared according to the following reaction scheme (Reaction scheme 3) and under the conditions listed in the following table (Table 3).

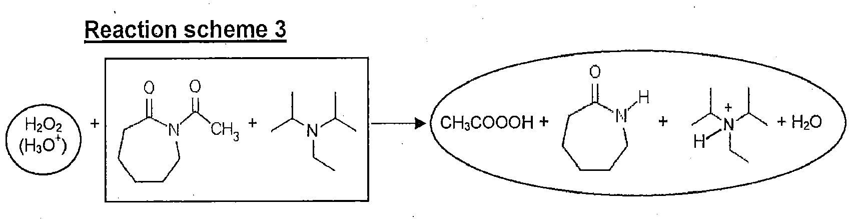

**Table 3**

| **Ingredient** | **% w/w** | **Chemical-physical properties** | **Quantity (g)** |
|---|---|---|---|
| **A: HYDROPHILIC component - Oxygen generator** | | | |
| Hydrogen peroxide | 19.50 | pH = 2.30 - 2.40 pH units at 20°C | 56.0 |
| Distilled water q.s. at | 100.00 | | |

| **LIPOPHILIC component - Donor of an acetyl group and tertiary amine** | | | |
|---|---|---|---|
| *N*-acetylcaprolactam | 94.00 | | 30.0 |
| *N,N,N*-diisopropylethylamine | 6.00 | | |

| **Peracetic acid solution + system amine/ammonium ion** | | | |
|---|---|---|---|
| Hydrogen peroxide | 7.0 - 8.5 | pH = 7.60 (T_{0 minutes}) - 6.40 (T_{5 minutes}) - 6.20 (T_{10 minutes}) - 4.85 (T_{30 minutes}) - 3.75 (T_{120 minutes}) | 86.0 |
| Peracetic acid | 5.0 - 6.0 | | |
| Caprolactam | 26.45 | | |
| System amine/ammonium ion | 2.09 | | |
| Distilled water q.s. at | 100.00 | | |

A solution of hydrogen peroxide was prepared at 19.5% w/w by diluting 31.2 g of hydrogen peroxide at 35% w/w with distilled water as needed at 56.0 g and the pH was determined at 20°C. A mixture of 28.20 g was prepared separately containing 28.20 g of N-acetylcaprolactam and 1.80 g of *N,N,N*-diisopropylethylamine and was then added under agitation to the hydrogen peroxide solution in order obtain a concentrated solution of peracetic acid having a concentration by weight between 5.0% and 6.0%. During the reaction, an increase in temperature of about 10°C was observed.

Both the pH variation over time of the peracetic acid solution and of the percentage concentration weight/ volume of peracetic acid thus obtained were determined, and the results are reported in Table 3 and in the following diagrams E and F.

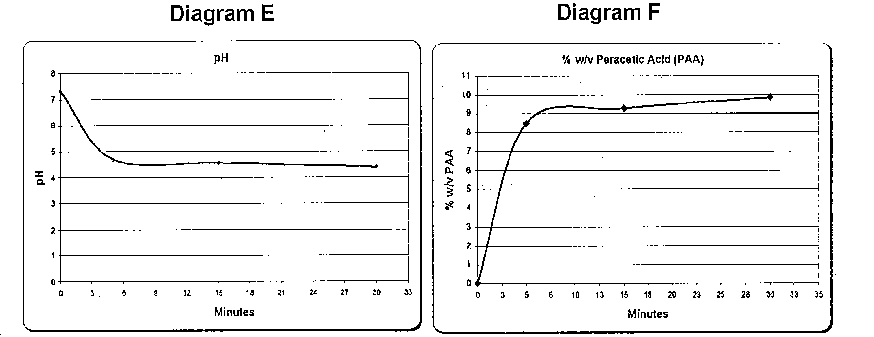

In diagram E one can note that, at the time of mixing the two components (t = 0), the acidic pH of the hydrogen peroxide solution instantly took a neutral basic value (7.0 - 8.0), which allows the formed hydroperoxide anions (HOO⁻) to effectively lead a nucleophilic attack to the carbonyl carbon of the acetyl group. Without this instantaneous increase in pH, the perhydrolysis reaction does not take place (as one can see in Example 4).

After the initial instant, the rapid formation of peracetic acid (as one can see in diagram F) is accompanied by an equally rapid decrease in pH down to acidic values within the range of 4.0 to 5.0, which allows the solution to preserve its stability.

### Example 4

### Test of preparation of a solution of peracetic acid in the absence of the tertiary amine N,N,N-diisopropylethylamine.

An attempt was made to prepare a concentrated solution of peracetic acid under the conditions described in the following table (Table 4), in which the isopropyl alcohol replaces the tertiary amine N,N,N-diisopropylethylamine used in Examples 1-3.

**Table 4**

| **Ingredient** | **%w/w** | **Chemical-physical properties** | **Quantity (g)** |
|---|---|---|---|
| **A: HYDROPHILIC component - Oxygen generator** | | | |
| Hydrogen peroxide | 19.50 | pH = 2.30- 2.40 pH units at 20°C | 56.0 |
| Distilled water q.s. at | 100.00 | | |

| **LIPOPHILIC component** - **Donor of an acetyl group and solvent/dispersant** | | | |
|---|---|---|---|
| *N*-acetylcaprolactam | 94.00 | | 30.0 |
| Isopropyl alcohol | 6.00 | | |

| **Mixture A+B** | | | |
|---|---|---|---|
| Hydrogen peroxide | 12.70 | pH = 2.40 (T_{0 minutes}) - 2.40 (T_{5 minutes}) - 2.40 (T_{10 minutes}) - 2.35 (T_{30 minutes}) - 2.35 (T_{120 minutes}) | 86.0 |
| Peracetic acid | 0.0 | | |
| Caprolactam/*N-*acetylprolactam | 32.80 | | |
| Isopropyl alcohol | 2.09 | | |
| Distilled water q.s. at | 100.00 | | |

A solution of hydrogen peroxide was prepared at 19,5% w/w by diluting 31.2 g of hydrogen peroxide at 35% w/w with distilled water as needed at 56.0 g and the pH was determined at 20°C. A mixture of 30.0 g was prepared separately containing 28.20 g of N-acetylcaprolactam and 1.80 g of isopropyl alcohol and was then added under agitation to the hydrogen peroxide solution. No increase in pH was detected, nor formation of peracetic acid. Indeed, the mixture took a milky appearance due to the insolubility of the *N*-acetylcaprolactam in the solution of hydrogen peroxide.

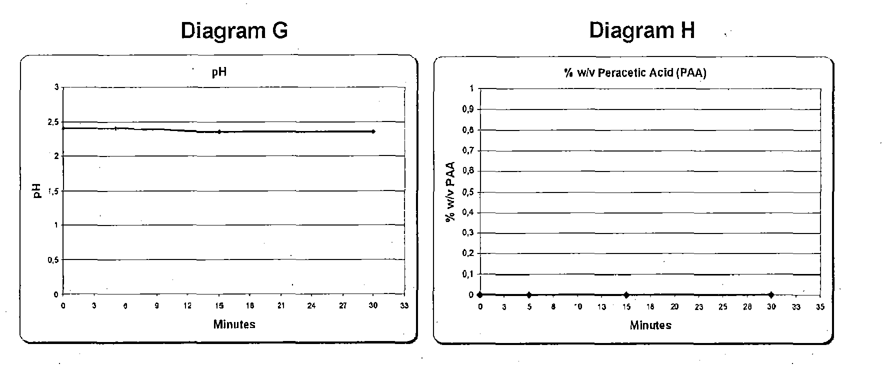

In diagram G one can note that, at the time of mixing the two components (t = 0), the acidic pH of the hydrogen peroxide solution remains unchanged, which does not allow the hydroperoxide anions (HOO⁻) to be formed and to effectively lead a nucleophilic attack to the carbonyl carbon of the acetyl group. Without the instantaneous increase in pH the perhydrolysis reaction cannot take place and consequently there is no formation of peracetic acid. Indeed, in the aqueous mixture with an acidic pH, hydrolysis prevails, with consequent formation of acetic acid instead of peracetic acid.

None of the pH regulators proposed sofar has the characteristics of aliphatic amines with weak or no nucleophilic character as provided in the composition of the lipophilic component of the system object of the present invention.

These characteristics, namely, weak or null nucleophilic character, lipophilic nature and capacity to subtract hydrogen ion (proton scavenger) from an aqueous acid in orderto raise the pH are essential elements for the pH regulator of a system or kit preferably comprising only two liquid components that are separately stored and stable over time and after being mixed quickly form a concentrated and diluted organic peroxyacid in aqueous or hydroalcoholic solution.

The inorganic regulators mentioned e. g. in US-2009/0043213 are, instead, exclusively soluble in water (by not being lipophilic), they all have nucleophilic activity, sometimes a strong one, and thus they cannot be mixed at all in a stable liquid form with the molecules of O-acyl and/or N-acyl donors, otherwise they would immediately degrade owing to breaking of the acyl bond. In fact, in that prior art document, there is provided to add them to the aqueous (hydrophilic) solution of hydrogen peroxide immediately before the addition of the acetic anhydride. As a matter of fact, should they be directly added to the acetic anhydride (typical example of a reactive O-acyl donor), there would occur an immediate hydrolysis reaction and subsequent formation of acetic acid instead of peracetic acid. Moreover, the addition of such pH regulators to the solution containing hydrogen peroxide is provided immediately before the addition of acetic anhydride, and certainly not long before (days or months), as it is known that raising the pH value of any solution of hydrogen peroxide will result in an increase of its reactivity and thus of its instability with subsequent loss of active oxygen.

The acid environment in which the liquid, hydrophilic and oxygen-liberating component of the system the subject ' matter of the present invention is kept, makes it possible to obtain greater stability compared to the above described state of the art even in adverse environmental conditions, and avoids the adoption of specific technical measures for transport and storage.

In so far as US-2005/109981 is concerned, it describes amines which differ from those of the present invention since they include nucleophilic functional groups. Such amines, mixed with the O-acetyl donor lipophilic component, acting as a corrosion inhibitor, do not ensure long-term stability, since the nucleophilic functional groups break the ester bond with consequent loss of active principle. Moreover, the inclusion of these amines in the hydrogen peroxide-based hydrophilic component of a hypothetical two-component system always involves instability due to pH being raised and requires an amount high enough to counteract over time the continuous pH lowering due to the peroxide degradation, which increases the amount of hydrogen ions in the solution. With a system in accordance with the present invention it is also possible to use a reduced quantity of pH regulator component in comparison with the solutions proposed up to date, since rise in pH is to be obtained only at the moment of mixing the two liquid components to cause the reaction of peracetic acid production. It should be noted that the function of adjusting the pH in orderto form peracetic acid according to document US-2005/109981 system is performed by the inorganic base included in the aqueous hydrogen peroxide solution. However, such inorganic base is a source of destabilization of the aqueous hydrogen peroxide owing to the increase in pH. For the stabilization thereof, it is necessary to take advantage of the typical solid state of percarbonate or perborate and others, or add this third pH regulating element to distilled water immediately before adding the lipophilic part A containing the bleach activator, thereby obtaining a three element system which is poorly practical in practice.

Patent application EP-1 371 643 does not teach at all a stable combination of an acetyl group donor and aliphatic amines with low or no nucleophilic character having the function of pH regulator, as in the present invention. Such prior document teaches only that the acetyl group donors should not be in aqueous solution or in mixtures with components having nucleophilic character, but, likewise US-2005/109981, suggests placing the pH regulator component in solution with the hydrophilic component (source of peroxide), which involves the same disadvantages as those described above, namely peroxide degradation and the need for a large amount of pH regulator component in order to achieve a sufficient rise in pH at the time of mixing together the hydrophilic and lipophilic components.

The solution obtained by the system and process according to the present invention, however, owing to a greater biocidal effect than the solutions proposed up to now, as the system amine/ammonium ion acts synergistically with peracetic acid, can be used as such, or after suitable dilution, as a disposable or reusable solution acting as disinfectant, sterilizer, bleach, descaler and detoxifier of industrial waste (e.g. cyanide and phosgene), with high performance in all fields in which such effects are required.

## Claims

1. A kit system of two liquid parts which are designed to produce extemporaneously, when mixed together, an aqueous or hydroalcoholic solution of a concentrated or dilute peracetic acid, said system including a lipophilic part and a hydrophilic part, with the lipophilic part including at least one donor of at least one acetyl group, whereas the hydrophilic part includes an aqueous or hydroalcoholic solution at acidic pH containing an oxygen donor,
said donor of at least one acetyl group being selected from N-acetylated and *O*-acetylated compounds, and
said aqueous or hydroalcoholic solution at acidic pH, containing an oxygen donor being an aqueous or hydroalcoholic solution of hydrogen peroxide at pH <5.5, and with a concentration by weight between 0.01% and 90%,
**characterized in that** said lipophilic part comprises at least one pH regulator/raiser including at least one aliphatic amine with weak or no nucleophilic character and free from nucleophilic functional groups, said aliphatic amine acting as pH raiser/regulator at the time of mixing together the two hydrophilic and lipophilic parts, thereby making it possible to obtain a fast and efficient production of peracetic acid,
said at least one aliphatic amine with weak or no nucleophilic character being selected from the group comprising at least one cyclical amine of Formula (II) 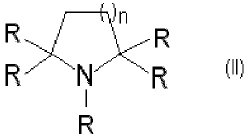 in which
R is hydrogen and/or methyl and/or an alkyl group,
n is 0, 1 or 2;
and/or at least a tertiary amine of Formula (III) 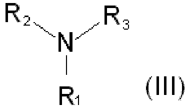 in which R₁, R₂ and R₃ are an alkyl group having a length of at least 2 carbon atoms.

2. A system according to claim 1, **characterized in that** said at least one cyclical amine of Formula (II) is selected from the group comprising 2,2,6,6-tetramethylpiperidine, 2,2,5,5-tetramethylpyrrolidine, 2,6-dimethylpiperidine, 2,5-dimethylpyrrolidine, *N-*ethylpyrrolidine and *N*-ethylpiperidine.

3. A system according to claim 1, **characterized in that** said at least one tertiary amine of Formula (III) is selected from the group comprising *N*,*N*,*N*-diisopropylethylamine, *N*,*N*,*N*-triethylamine, *N,N,N-*triisopropylethylamine, *N*,*N*,*N*-diisopropylbutylamine.

4. A system according to any preceding claim, **characterized in that** said at least one pH regulator is present in the lipophilic component at a concentration by weight from 0.1% to 10.0% w/w.

5. A system according to any preceding claim, **characterized in that** said *N*-acetylated compounds are selected from the group comprising *N*,*N*,*N*',*N*'-tetraacetylethylenediamine, tetraacetylglycoluril, at least one (C₄-C₁₀) *N*-acetyllactam and at least one *N*-acetylimide having a number of carbon atoms greater than or equal to 4.

6. A system according to claim 5, **characterized in that** said at least one (C₄-C₁₀) *N*-acetyllactam comprises *N*-acetylcaprolactam.

7. A system according to claim 5, **characterized in that** said at least one *N*-acetylimide having a number of carbon atoms greater than or equal to 4 comprises *N*-acetylsuccinimide, *N*-acetylphthalimide and 2-*n-*nonyl-*N*-acetylsuccinimide.

8. A system according to any preceding claim, **characterized in that** said *O*-acetylated compounds are selected from the group comprising acetic anhydride, acetylsalicylic acid and acetate esters of sugars, of glycols and polyols.

9. A system according to claim 8, **characterized by** in said acetate esters of sugars, glycols and polyols, comprise pentaacetate glucose, octaacetate sucrose, esaacetate mannitol, diacetate glycerol and diacetate propylene glycol.

10. A system according to any preceding claim, **characterized in that** said aqueous or hydroalcoholic solution at acidic pH, containing an oxygen donor, is an aqueous or hydroalcoholic solution of hydrogen peroxide at pH <5.5, and with a concentration by weight between 3% and 35%.

11. A system according to any preceding claim, **characterized by** adding at least one chemical additive to one of the two components of said system according to any claim from 1 to 10, said at least one chemical additive being selected from the group including at least one alcohol, at least one surfactant agent, at least one dispersing agent, at least one dye, at least one anti-corrosion agent, at least one diol and at least one polyol.

## Patentansprüche

1. Ein Kitsystem zweier flüssiger Teile, die, wenn sie miteinander vermischt werden, zur unmittelbaren Erzeugung einer wässrigen oder hydroalkoholischen Lösung einer konzentrierten oder verdünnten Peressigsäure bestimmt sind, wobei das besagte System einen lipophilen Teil und einen hydrophilen Teil aufweist, mit dem lipophilen Teil zumindest umfassend einen Donor einer mindestens einen Acetylgruppe, worin der hydrophile Teil eine wässrige oder hydroalkoholische Lösung bei saurem pH-Wert umfasst, enthaltend einen Sauerstoffdonor,
der besagte Donor der mindestens einen Acetylgruppe ausgewählt ist aus N-acetylierten und O-acetylierten Verbindungen, und
die besagte wässrige oder hydroalkoholische Lösung bei saurem pH-Wert, enthaltend einen Sauerstoffdonor, eine wässrige oder hydroalkoholische Lösung von Wasserstoffperoxid bei einem pH-Wert < 5,5 und mit einer Gewichtskonzentration zwischen 0,01 % und 90 % ist,
**dadurch gekennzeichnet, dass** der besagte lipophile Teil mindestens einen pH-Regulator/-Heber umfasst, umfassend mindestens ein aliphatisches Amin mit schwachem oder keinem nukleophilen Charakter und frei von nukleophilen funktionellen Gruppen, das besagte aliphatische Amin als pH-Heber/Regulator zum Zeitpunkt des Vermischens der beiden hydrophilen und lipophilen Teile wirkt, wodurch eine schnelle und effiziente Herstellung von Peressigsäure ermöglicht wird,
das besagte mindestens eine aliphatische Amin mit schwachem oder keinem nukleophilen Charakter ausgewählt ist aus der Gruppe, die mindestens ein zyklisches Amin der Formel (II) aufweist, 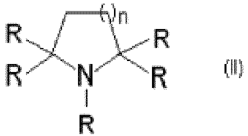 in der
R Wasserstoff und/oder Methyl und/oder eine Alkylgruppe ist,
n 0, 1 oder 2 ist;
und/oder mindestens ein tertiäres Amin der Formel (III), 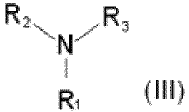 in der Ri, R₂ und R₃ eine Alkylgruppe mit einer Länge von mindestens 2 Kohlenstoffatomen sind.

2. System nach Anspruch 1,
**dadurch gekennzeichnet, dass** mindestens ein zyklisches Amin der Formel (II) ausgewählt ist aus der Gruppe umfassend 2,2,6,6-Tetramethylpiperidin, 2,2,5,5-Tetramethylpyrrolidin, 2,6-Dimethylpiperidin, 2,5-Dimethylpyrrolidin, *N-*Ethylpyrrolidin und *N*-Ethylpiperidin.

3. System nach Anspruch 1, **dadurch gekennzeichnet, dass** mindestens ein tertiäres Amin der Formel (III) ausgewählt ist aus der Gruppe umfassend *N*,*N*,*N-*Diisopropylethylamin, *N*,*N*,*N*-Triethylamin, *N*,*N*,*N*-Triisopropylethylamin, *N*,*N*,*N-*Diisopropylethylamin.

4. System nach irgendeinem vorangehenden Anspruch, **dadurch gekennzeichnet, dass** mindestens ein pH-Regulator in der lipophilen Komponente mit einer Gewichtskonzentration von 0,1 % bis 10,0 % w/w vorhanden ist.

5. System nach irgendeinem vorangehenden Anspruch, **dadurch gekennzeichnet, dass** *N*-acetylierte Verbindungen ausgewählt sind aus der Gruppe umfassend *N*,*N*,*N'*,*N'-*Tetraacetylethylendiamin, Tetraacetylglykoluril, mindestens ein (C₄-C₁₀) *N-*Acetyllactam und mindestens ein *N*-Acetylimid mit einer Anzahl von Kohlenstoffatomen, die größer als oder gleich 4 ist.

6. System nach Anspruch 5, **dadurch gekennzeichnet, dass** mindestens ein (C₄-C₁₀) *N-*Acetyllactam *N*-Acetylcaprolactam umfasst.

7. System nach Anspruch 5, **dadurch gekennzeichnet, dass** mindestens ein *N*-Acetylimid mit einer Anzahl von Kohlenstoffatomen, die größer als oder gleich 4 ist, *N-*Acetylsuccinimid, *N*-Acetylphthalimid und 2-*n*-nonyl-*N*-Acetylsuccinimid aufweist.

8. System nach irgendeinem vorangehenden Anspruch, **dadurch gekennzeichnet, dass** *O*-acetylierte Verbindungen ausgewählt sind aus der Gruppe umfassend Essigsäureanhydrid, Acetylsalicylsäure und Acetatester von Zuckern, von Glykolen und Polyolen.

9. System nach Anspruch 8, **dadurch gekennzeichnet, dass** die besagten Acetatester von Zuckern, Glykolen und Polyolen Glucosepentaacetat, Saccharoseoktaazetat, Mannitolhexaacetat, Glyceroldiacetat und Propylenglykoldiacetat umfassen.

10. System nach irgendeinem vorangehenden Anspruch, **dadurch gekennzeichnet, dass** die besagte wässrige oder hydroalkoholische Lösung bei saurem pH-Wert, enthaltend einen Sauerstoffdonor, eine wässrige oder hydroalkoholische Lösung von Wasserstoffperoxid bei einem pH-Wert < 5,5 und mit einer Gewichtskonzentration zwischen 3 % und 35 % ist.

11. System nach irgendeinem vorangehenden Anspruch, **dadurch gekennzeichnet, dass** durch Zugabe von mindestens einem chemischen Zusatzstoff zu einer der beiden Komponenten des besagten Systems nach irgendeinem vorangehenden Anspruch 1 bis 10 der besagte mindestens eine chemische Zusatzstoff ausgewählt ist aus der Gruppe, die mindestens einen Alkohol, ein oberflächenaktives Mittel, mindestens ein Dispersionsmittel, mindestens einen Farbstoff, mindestens ein Korrosionsschutzmittel, mindestens ein Diol und mindestens ein Polyol umfasst.

## Revendications

1. Un kit système de deux parties liquides qui sont désignées pour produire extemporanément, lorsqu'elles sont mélangées, une solution aqueuse ou hydro alcoolique de acide peracétique concentré ou dilué, ledit système comprenant une partie lipophile et une partie hydrophile, avec la partie lipophile comprenant au moins un donneur d'au moins un groupe acétyle, tandis que la partie hydrophile comprend une solution aqueuse ou hydro alcoolique à pH acide contenant un donneur d'oxygène,
ledit donneur d'au moins un groupe acétyle étant choisi dans les composés N-acétylés et O-acétylés, et
ladite solution aqueuse ou hydro alcoolique à pH acide, contenant un donneur d'oxygène étant une solution aqueuse ou hydro alcoolique de peroxyde d'hydrogène à pH <5,5 et avec une concentration pondérale comprise entre 0,01% et 90% **caractérisé en ce que** ladite partie lipophile comprend au moins un régulateur/élévateur de pH comprenant au moins une amine aliphatique ayant un caractère nucléophile faible ou nul et sans des groupes fonctionnelles nucléophiles, ladite amine aliphatique agissant comme élévateur/régulateur de pH au moment du mélange ensemble des deux parties hydrophiles et lipophiles, permettant ainsi d'obtenir une production rapide et efficace d'acide peracétique,
ladite au moins une amine aliphatique ayant un faible ou nul caractère nucléophile étant choisie dans le groupe comprenant au moins une amine cyclique avec la formule (II) 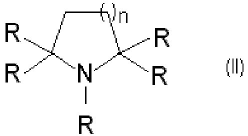 dans laquelle
**R** est l'hydrogène et/ou le méthyle et/ou un group alkyle
**n** est 0, 1 ou 2 ;
et/ou au moins une amine tertiaire avec la formule (III) 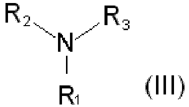 dans laquelle Ri, R₂ et R₃ sont un alkyle groupe ayant une longueur d'au moins 2 atomes de carbone.

2. Un système selon la revendication 1, **caractérisé en ce que** ladite au moins une amine cyclique avec la formule (II) est choisie dans le groupe comprenant 2,2,6,6-tétraméthylpipéridine, 2,2,5,5-tétraméthylpyrrolidine, 2, 6-diméthylpipéridine, 2,5-diméthylpyrrolidine, N-éthylpyrrolidine et N-éthylpipéridine.

3. Un système selon la revendication 1, **caractérisé en ce que** ladite au moins une amine tertiaire avec la formule (III) est choisie dans le groupe comprenant N,N,N-diisopropyléthylamine, N,N,N-triéthylamine, N,N,N- Triisopropyléthylamine, N,N,N -diisopropylbutylamine.

4. Un système selon l'une quelconque des revendications précédentes, **caractérisé en ce que** ledit au moins un régulateur de pH est présent dans le composant lipophile à une concentration pondérale comprise entre 0,1% et 10,0% p/p.

5. Un système selon l'une quelconque des revendications précédentes, **caractérisé en ce que** lesdits composés N-acétylés sont choisis dans le groupe comprenant N,N,N',N'-tétraacétyléthylènediamine, tétraacétylglycoluril, au moins un (C₄-C₁₀) N-acétylactame et au moins un N-acétylimide ayant un nombre d'atomes de carbone supérieur ou égal à 4.

6. Un système selon la revendication 5, **caractérisé en ce que** ledit au moins un (C₄-C₁₀) N-acétylactame comprend N-acétylcaprolactame.

7. Un système selon la revendication 5, **caractérisé en ce que** ledit au moins un N-acétylimide ayant un nombre d'atomes de carbone supérieur ou égal à 4, comprend N-acétylsuccinimide, N-acétylphtalimide et 2-n-nonyl-N-acétylsuccinimide.

8. Un système selon l'une quelconque des revendications précédentes, **caractérisé en ce que** lesdits composés O-acétylés sont choisis dans le groupe comprenant l'anhydride acétique, l'acide acétylsalicylique et les esters d'acétate des sucres, des glycols et des polyols.

9. Un système selon la revendication 8, **caractérisé en ce que** lesdits esters d'acétate des sucres, des glycols et des polyols comprennent le glucose pentaacétate, le saccharose octaacétate, le mannitol esaacétate, le glycérol diacétate et le propylène glycol diacétate.

10. Un système selon l'une quelconque des précédentes revendications, **caractérisé en ce que** ladite solution aqueuse ou hydro alcoolique à pH acide, contenant un donneur d'oxygène, est une solution aqueuse ou hydro alcoolique de peroxyde d'hydrogène à pH < 5,5 et avec une concentration pondérale comprise entre 3% et 35%.

11. Un système selon l'une quelconque des revendications précédentes, **caractérisé en ce que** l'on ajoute au moins un additif chimique à l'un des deux composants dudit système selon l'une quelconque des revendications de 1 à 10, ledit au moins un additif chimique étant choisi parmi le groupe comprenant au moins un alcool, au moins un agent tensioactif, au moins un agent dispersant, au moins un colorant, au moins un agent anticorrosion, au moins un diol et au moins un polyol.
